# EUROPEAN PATENT SPECIFICATION

(11) **EP 2 926 326 B1**
(45) Date of publication and mention of the grant of the patent: **07.11.2018**
(21) Application number: 12889583.6
(22) Date of filing: 03.12.2012
(51) Int. Cl.: G08B 21/04, G08B 25/01, A61B 5/00, F25D 29/00

(54) **ARRANGEMENT AND METHOD FOR NUTRITION AND CARE SERVICES**
ANORDNUNG UND VERFAHREN FÜR ERNÄHRUNGS- UND PFLEGEDIENSTE
DISPOSITIF ET PROCÉDÉ POUR SERVICES DE NUTRITION ET DE SOINS

(43) Date of publication of application: 07.10.2015
(73) Proprietor: Menumat OY, 00880 Helsinki (FI)
(72) Inventor: HAUTALA, Pekka, FI-Espoo 02170 (FI); PENTTINEN, Jukka, FI-Helsinki 00330 (FI)
(74) Representative: Salomäki, Juha Kari Ensio
(86) International application number: PCT/FI2012/051202
(87) International publication number: WO 2014/087040

(56) References cited:
- EP-A2- 2 472 487
- WO-A1-03/044755
- WO-A1-2005/027488
- WO-A1-2005/033598
- WO-A1-2009/125069
- WO-A1-2009/125069
- US-A- 5 673 304
- US-A1- 2004 030 531
- US-A1- 2008 001 735
- US-A1- 2011 291 827

## Description

The object of the invention is an arrangement as defined in the preamble of claim 1 and a method as defined in the preamble of claim 10 for nutrition and care services.
In this application the nutrition and care services mean nutrition, such like acquiring foodstuffs and processing them, and in cases of emergency making fast and easy alarms possible.
Conventionally, the processing of foodstuffs, especially of foodstuffs for a home, comprises a shopping trip to a store, the storing of the foodstuffs in a refrigerator or deep-freezer, and the preparation of food e.g. on a cooker ring, in the oven or in a microwave oven. In practice a lot of time is spent on collecting foodstuffs and correspondingly the processing of foodstuffs for preparing food requires a lot of different apparatuses and utensils that take a lot of space and also form complex entities that are, among other things, difficult to maintain owing to their different natures. In addition there are many groups of people, such as elderly people, handicapped people and also children, who are unable or incompetent to themselves prepare their food portions using current methods and apparatuses.
Various food automats, which are able to prepare food by heating pre-frozen portions, have been developed to rectify the aforementioned nutrition problem. For example, the Swedish patent publication no. SE 459618 discloses a food automat, which prepares various food portions according the choice of the user. A problem with this apparatus, however, as with other corresponding prior-art apparatuses is, among others, the extremely complex construction, which makes the apparatus expensive and one that requires a lot of maintenance, although despite this susceptible to malfunction. The apparatus comprises, among other things, electric motors and transporter belts, which comprise a lot of moving parts, which are additionally in demanding conditions in the cold space and can therefore easily become unserviceable.

Generally speaking, apparatuses according to prior art, in which the storage, transfer and heating of food portions is integrated into one, thus have as a problem the magnitude of the manufacturing costs of the apparatuses and consequently an expensive purchase price, and also that the technical operating reliability has, in practice, been poor owing to the complex construction.

Some groups of people, for instance elderly people and handicapped people, who need a food automat for their nutrition may also need a safety device to make an alarm if something happens to them while they are at home alone. This kind of device can comprise for instance an alarm button worn around a wrist, which alarm button is connected wirelessly to a base unit located at home. The base unit has further connection for getting help. The connection can be for instance a standard telephone line or a GSM-line or a corresponding connection. For instance if an elderly person falls at home, he or she can send an alarm by pressing the alarm button worn around the wrist.

The alarm can also be automated in a way that if a person falls down, an alarm is sent automatically. This kind of automated device comprises fall detector or detectors, which can be for instance optical sensors or acceleration sensors.

These kinds of devices are known in prior art. The problem is that some groups of people may have many different apparatuses and devices at their homes, which is complicated and also expensive, and there is a danger to lose one or more of the several devices.

Patent publications WO2009/125069 A1 and WO2005/033598 A1 present prior art technology in the same field of technology as the present invention. However, they do not mention an alarm system that is integrated into the control system of the food automat.

The object of the present invention is to eliminate the aforementioned drawbacks and to achieve an operationally reliable, and as service-free as possible, arrangement and method for nutrition and care services including for example the storage, heating and serving of food portions. Daily food portions can be prepared with the apparatus according to the invention in both homes and institutions as well as in small offices where food is eaten, thereby considerably reducing the amount of work needed. Preparing daily food portions will be easier and will also become possible for people who cannot prepare their food themselves, or are unwilling or incompetent to do so. Another object of the present invention is to integrate an alarm system to the apparatus that stores and heats food, and to use the same connections for the alarm operations as for the food handling operations. In order to achieve these objectives, the arrangement of the invention is characterized by what is disclosed in the characterization part of claim 1. Correspondingly, the method according to the invention is characterized by what is presented in the characterization part of claim 12. Other preferred embodiments of the invention are characterized by what is presented in the other claims.

One significant advantage of the arrangement and the method according to the invention - or more briefly and in other words, of the solution - with respect to the food automats of prior art is that the manufacturing costs will decrease, in which case the price of the apparatus will decrease and the technical operating reliability will nevertheless at the same time improve. In this case by means of the arrangement all groups of people are provided with an easy and effortless as well as a labour-saving, space-saving, and economical method for foodstuff supply in the home, in institutions and also in small offices. In this case elderly people, handicapped people and also children can easily prepare food themselves and can themselves decide what they eat in their homes and when they eat.

Another advantage of the solution according to the invention is that monitoring of the nutrition level is easy to implement, in which case users can themselves regulate the most pleasing and healthy diet for them. Nutrition intake, consumption habits, costs and other related aspects can be regularly monitored using the information collected in the database of the apparatus, and if desired reports can be printed with a printer connected to the apparatus.

In addition, the solution according to the invention comprises the possibility, depending on the application, to maintain a connection via a telecommunication network or other suitable communication network with a supplier of foodstuffs or with some other organizations or persons, such as relatives, and also to monitor the user of the apparatus via an information network. In addition, different instructions can be given and a program, e.g. as entertainment, can also be sent via the network.

Yet another advantage of the solution according to the invention is that an alarm system is integrated to the apparatus that stores and heats food and the alarm system uses the same communication means that the apparatus. Thus there is no need for separate apparatus and communication means, which makes the overall costs smaller, and also makes the use of the alarm system easier and more simple.

In the following, the invention will be described in more detail by the aid of one preferred embodiment with reference to the attached drawings, wherein
- Fig. 1: diagrammatically presents a total solution according to the invention,
- Fig. 2: presents a partly simplified front view of the cold-storage space of the arrangement according to the invention with the door removed,
- Fig. 3: presents an oblique top view of the oven of the arrangement according to the invention,
- Fig. 4: presents an oblique top view of a storage shelf intended for a food portion of the arrangement according to the invention,
- Fig. 5: presents a top view of the storage shelf according to Fig. 4 when empty,
- Fig. 6: presents a top view of the storage shelf according to Fig. 4 with a food portion,

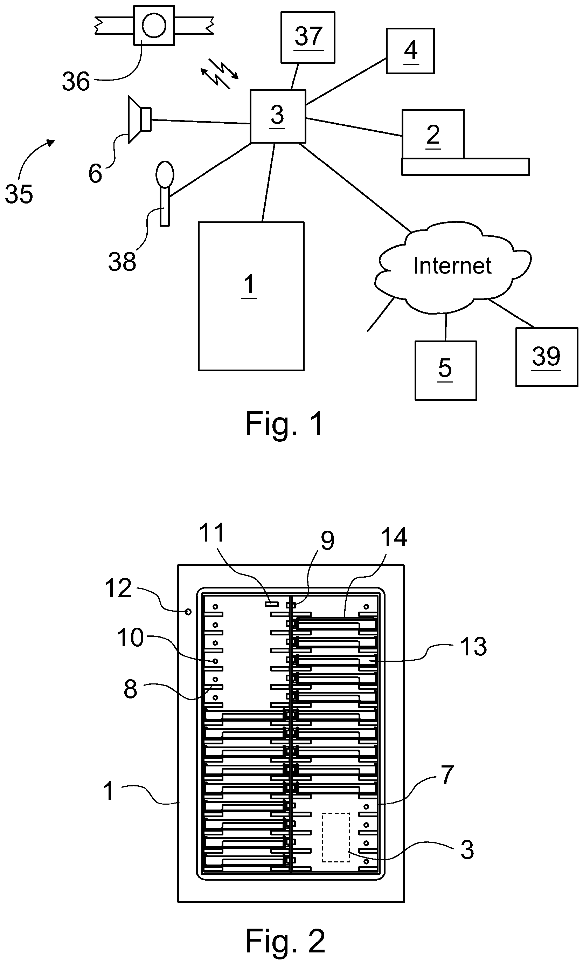

The solution of the invention according to Fig. 1 comprises at least a deep-freezer or refrigerator that functions as a cold-storage space 1, a circulating-air oven or corresponding that functions as a heating means 2, a control system 3 comprising at least a computer or microprocessor, one or more user interfaces 4, a system server 5, and also an alarm system 35.

The ready, pre-frozen food portions are placed for storage into the deep-freezer that functions as a cold-storage space 1, which deep-freezer is at a suitably low temperature for deep-frozen storage. Alternatively, the ready, pre-cooled food portions are placed for storage into the refrigerator that functions as a cold-storage space 1, which refrigerator is at a suitable temperature for refrigerated storage. In one preferred embodiment of the invention all the food portions in the apparatus are stored at the same temperature, but there can also be differences in the storage temperatures. The circulating-air oven that functions as a heating means 2 is fitted to heat a food portion to an even temperature that is suitable from the viewpoint of the user either by means of resistance heating technology, microwave heating technology, or a combination of these, or by means of some other heating technology suited to the purpose.

The user interface 4 is e.g. connected to the control system 3 of the apparatus according to the arrangement, which control system comprises the programs and actuators needed by the apparatus. The control system 3, which functions as the data processing part, is connected to the cold-storage space 1 in order to e.g. track, monitor and control the functions and temperature of the cold-storage space 1. The control system 3 is further connected to the heating means 2, in order to e.g. track, monitor and control the functions and temperature of the heating means 2, and to the alarm system 35 in order to make it possible to use the same food processing apparatus for sending an emergency alarm and other information, and for communicating questions and/or instructions to the person using the alarm system 35.

The same control system 3 can also be connected to more than one cold-storage space 1 and heating means 2, and when necessary, to more than one alarm system 35. For example, in some big institutions, there can be one or more cold-storage spaces 1, heating means 2 and/or alarm systems 35, which are connected to the one and the same control system 3.

The control system 3 is also connected to a public telecommunication network, such as e.g. telephone network and/or the Internet or some other data communications network. The connection can be wireless or via wire. The control system 3 comprises at least a processor part, a data communication part, which comprises the necessary data communication functions, a control part, which comprises the necessary control functions, monitoring functions and regulation functions, and also connection means for receiving and sending information.

Telecommunications can be used for ordering new foodstuffs from the foodstuffs supplier. This function can be automatic, in which case a new order is placed when the foodstuffs decrease below the minimum set quantity. The order can also be manual, in which case the user defines the quantity and type of the foodstuffs by means of the user interface 4 of the apparatus. By means of the network interface it is also possible to transmit fault information about any defects that possibly occur in the apparatus and alerts about depletion of the food, and also to send data to be heard from the loudspeaker 6 of the apparatus, which is connected to the control system 3 and placed in connection with the heating means 2, e.g. on the base of the heating means 2. For tracking and monitoring the apparatus and the use of the apparatus e.g. a phone can be used, by means of text messages, or a second computer, from which also the content and quantity of food portions of an apparatus in the placement location can be viewed. This type of external computer can be e.g. a system server 5 connected to the network.

The control system 3 further offers the possibility of controlling the nutritional amounts and quality of the foodstuffs, either automatically or by guiding the user. Monitoring of the nutrition level can be easily implemented, in which case if the user needs a diet the control system 3 ensures that it is implemented.

The deep-freezer or refrigerator that functions as a cold-storage space 1 can be freely positioned, even in another space with respect to the heating means 2, because the connection between the control system 3 of the cold-storage space 1 and the heating means 2 is wireless, e.g. a Bluetooth, infrared, radio or some other corresponding wireless connection. Both the control system 3 of the cold-storage space 1 and the heating means 2 comprise appropriate connection means for the connection. The connection between the control system 3 of the cold-storage space 1 and the heating means 2 can also be wired. Additionally, there can also be two or more units of the heating means 2 and two or more cold storage units, all of which comprise a connection to the control system 3. In this case a number of food portions can be prepared simultaneously.

The identification of food portions is based on a barcode system, the advantage of which are such clear markings that the arrangement according to the invention reliably identifies the foodstuffs brought into the apparatus, which e.g. the distribution vehicle of the food shop or other service organization brings on the basis of an order directly to the user of the apparatus. Other possible identification systems can also be used, such as RFID, for example.

The alarm system 35 comprises at least a portable alarm device 36, which is connected to the control system 3. The alarm device 36 is e.g. a button worn around the user's wrist and it is connected to the control system 3 wirelessly. The control system 3 is connected via the telecommunication network to a call center 39 such as a service center or relatives or corresponding. In this example the call center 39 is a separate service center. If, for instance, an accident happens, the user can press the button of the portable alarm device 36, in which case an emergency alarm is sent via the control system 3 and the telecommunication network to the service center 39, which can then send help.

The portable alarm device 36 can also be an automated device that detects if the user falls down, in which case an emergency alarm is sent automatically to the service center 39. This kind of automated alarm device can be equipped e.g. with optical sensors or acceleration sensors or corresponding equipment to detect downfalls. Also other automated alarms can be joined to the alarm system 35, for example measurements of heart pulse, body temperature, blood sugar value, etc. Automated alarm devices are connected to the control system 3 either with wire or wirelessly. An example of the alarm device connected to the control system 3 with wire is shown with a reference number 37 in Fig. 1.

The control system 3 comprises means to communicate with the portable alarm device 36 and with the user of the alarm device 36, and means to control the alarm device. For instance the control system 3 keeps track of the battery life of the portable alarm device 36 and informs if the battery voltage is low and the battery needs to be changed.

The alarm system 35 comprises also a microphone 38, which is able to catch voice from a wide distance. Like the loudspeaker 6, the microphone 38 is connected to the control system 3 and can be placed for example in connection with the heating means 2, e.g. on the base of the heating means 2. When an alarm has been made, the user who has e.g. fallen down and made the alarm, can talk with the people in the service center 39 via the microphone 38 and the loudspeaker 6. For instance, the user can tell his/her location in the house/apartment and what has happened, or if he/she has pressed the alarm button accidentally he/she can tell that no help is needed.

The alarm system 35 can also comprise one or more buttons, for instance in the user interface 4, which buttons can be used to send information to the service center 39. In case the user is not able to speak, he/she can e.g. send information of false alarm by pressing a button, which is meant for that.

Additionally, the alarm system 35 can comprise means to monitor the possible door sensors of the accommodation. In that case it is possible to know for instance, if the user of the alarm system has gone out.

The alarm system 35 may also have a bi-directional self-diagnostic function. The control system 3 can check the operation of the alarm device 36 or the wrist button and other wireless or wired sensors and also the alarm device 36 and sensors have a self diagnostic capability - they can send an error message to the control system 3 if they have noticed any malfunctioning.

Fig. 2 presents the cold-storage space 1 of the arrangement according to the invention, as viewed from the front and with the door removed. The cold-storage space 1 is in principle a conventional deep-freezer, inside which is fitted a shelving structure 7 provided with side walls and rear walls, which shelving comprises a plurality of shelf spaces 9a provided with support means 8 and guide means 9, in which shelf spaces are stored shelves 13 that are empty or that contain food portions 14 and that function as the storage means of food portions 14. In Fig. 2 some of the shelf spaces 9a are left empty for the sake of clarity and a food portion 14 is visible on only one topmost shelf 13. At the point of each shelf space 9a on the rear wall of the shelving structure is a switch 10, which is fitted to indicate whether a food portion 14 is on the shelf 13 or not. The cold-storage space 1 also comprises a door switch 12, which is fitted to monitor the opening and closing of the door, as well as a temperature sensor 11 for monitoring the temperature of the cold storage of the cold-storage space 1. In addition, at least the main components of the control system 3 are disposed in the compressor part of the cold-storage space, where they are in a safe place and out of the way.

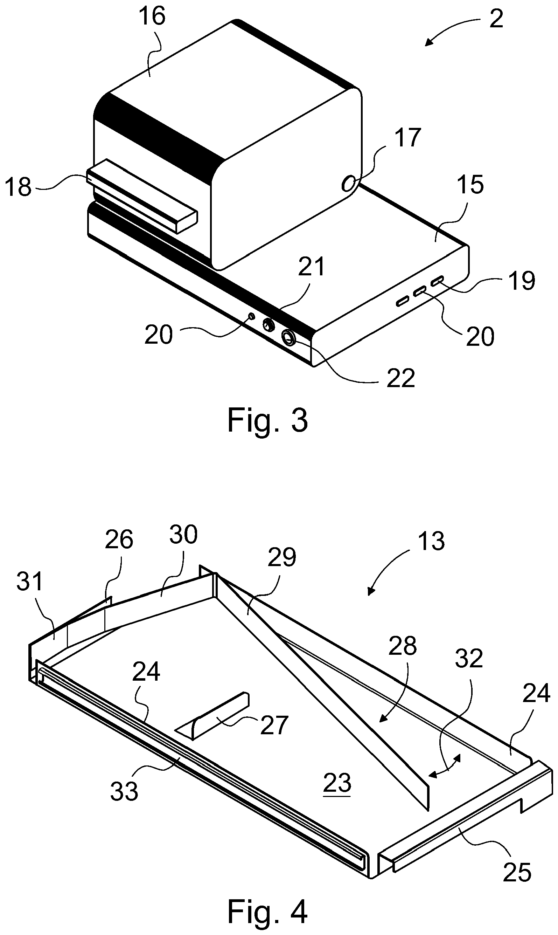

Fig. 3 presents the circulating-air oven that functions as the heating means 2 of the arrangement according to the invention. The oven comprises a frame part 15 and a hood 16 that is open at its base and hinged to the frame part with a hinge 17, which hood is fitted to turn to the open position from the front upwards by lifting it with the handle 18. The heating elements of the oven are inside the hood 16. Either one or a number of food portions can be heated with the oven at one and the same time. The frame part of the oven comprises a plurality of parallel connectors 19, which can be e.g. USB connectors or corresponding. A bar code reader 4a can be connected as one user interface to one connector 19 and e.g. also a USB memory stick 4b can be connected as a user interface to a second connector 19. Additionally, the frame part comprises a signal lamp 20, a pushbutton 21 and an iButton reader 22 that in a way also function as a user interface. The signal lamp 20, e.g. a LED light, indicates the functioning of the oven. For example, it is unlit in its normal state, flashes during heating of the oven and is continuously lit when the food is ready. The pushbutton 21, which is an Information button, can be pressed at any time whatsoever, in which case the apparatus announces in speech what it is doing or when it will be ready or what the situation is. For example, when pressing the pushbutton 21 in the heating phase the apparatus announces in speech how much heating time remains. In another phase the apparatus announces the information of the phase in question.

Correspondingly, an iButton reader is connected to escort memories, into each of which some code, e.g. the letters and numbers A1, A2, B3, etc, is programmed. Commands can be transmitted to the control system 3 with the escort memory and via the control system to the system server 5, in which case different commands are given with escort memories of different colors. For example, by means of escort memories a person can listen to his/her own voice mailbox, can choose whether to listen to radio broadcasts or not, and also can send a call request e.g. to his/her relative, in which case the system sends a call request by text message to a preselected mobile phone number, etc.

Fig. 4 presents a storage shelf 13 intended for a food portion 14 of the arrangement according to the invention. The shelf 13 is essentially the shape of a right-angled box and it contains an essentially flat base 23, side walls 24, a front wall 25 forming a handle, and a rear wall 26, which extends from the second side edge of the shelf 13 to approximately the midpoint of the shelf 13 in the width direction. In addition, the outer surface of one side wall 24 comprises a guide rail 33, which can be fitted into the guide means 9 in the shelving structure 7. A mechanical, spring-like sensing means 28 is fixed to the rear wall 26 of the shelf 13, which sensing means comprises at least a fixing part 31, a presser part 30 disposed in connection with it, and a sensor part 29 disposed in connection with the presser part 30, which sensor part is at an angle of essentially 90° with respect to the presser part. The sensing means 28 together with the parts are a spring element that is a flat bar in its shape, the presser part 30 of which is disposed at the point of the open rear end of the shelf 13, where there is no rear wall 26. At the bottom of the shelf 13 there is also a limiting means 27, which is fitted to support a food portion 14 disposed on the shelf 13 and to keep the food portion in the front part of the shelf.

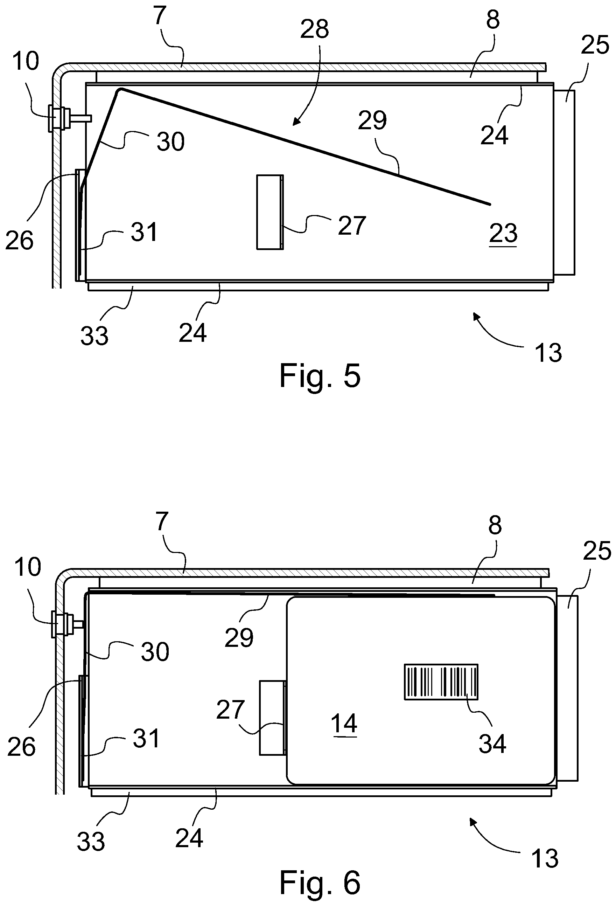

Figs. 5 and 6 present a top view of the storage shelf according to Fig. 4. In the situation according to Fig. 5 the shelf 13 is empty, in which case the spring-like sensing means 28 is free and turned under the force of the spring force to such a position that the sensor part 29 is in the at least partly empty place of the food portion, i.e. in the middle of the front part of the shelf. At the same time the presser part 30 is turned towards the inside of the shelf 13 such that when the empty shelf 13 is in its position in the shelf space 9a, the switch 10 on the rear wall of the shelving structure 7 is in the pushed-out position, in which case the control system 3 has received information that the shelf 3 that was pushed inside is empty.

Correspondingly, in the situation according to Fig. 6 the food portion 14 is placed on the front part of the shelf 13. The sensing means 28 is implemented such that when placing a food portion 14 into its position, the sensor part 29 of the sensing means 28 must be turned to the side towards the second side wall 24, in which case it remains between the food portion 14 and the side wall 24. At the same time the presser part 30 turns backwards so much that when pushing the shelf 13 into its position after filling the presser part 30 presses the switch 10 to the inner position, in which case the control system 3 of the guide receives information that the shelf 13 that was pushed inside contains a food portion 14. A bar code 34 is on the lid of the food portion 14 for identifying the food portion.

In the arrangement according to the invention deep-frozen food portions 14 are filled into the cold-storage space 1 of the apparatus e.g. such that at first a memory stick 4b and a barcode reader 4a are fixed into the USB connectors 19 for entering information relating to the food portions into the control system 3 of the apparatus. The memory stick 4b contains, among other things, the identification code of each food portion 14, both the written and the spoken name of the food portion, and also the heating instruction of the food portion. The barcode 34 of the food portion 14 is read with the reader 4a of the barcode before placing the food portion into the shelf space 9a of the cold-storage space 1. The food portion 14 is put on any empty shelf 13 whatsoever, which is then pushed into its position in an empty shelf space 9a of the shelving structure 7. When the shelf 13 is pushed to the bottom in the shelf space 9a, the switch 10 triggers and the guide of the apparatus detects that a food portion came into the shelf space 9a. Thus the control system 3 of the apparatus receives information about into which shelf space 9a the food portion came, and at the same time information at least about what the name and the heating instruction of the food portion 14 are.

When the user of the apparatus wants to have a meal, he/she selects the desired food portion from the menu, which is also provided with the numbers of the food portions. The front edge of the shelves 13 comprise corresponding numbers and the user starts the apparatus by opening the door of the cold-storage space 1, in which case the door switch detects that the door has opened and sends information about it to the control system 3 of the apparatus. After this the user pulls the shelf 13 that is provided with the number he/she selected out of the shelf space 9a, takes the food portion from the shelf 13, pushes the shelf 13 back into its position and closes the door of the cold-storage space 1, in which case the door switch 12 of the apparatus again reacts. Pulling out the shelf 13 simultaneously causes triggering of the switch 10, in which case the control system 3 receives information about the taking of the food portion 14 and announces the name of the food portion aloud via the loudspeakers 6.

Next the user puts the food portion 14 into the heating means 2 inside the hood 16. The control system 3 has information about the taking of the food portion from the cold-storage space 1, so the control system 3 monitors that the food portion is put into the heating means 2 within an appropriate time. The heating space of the heating means 2 comprises a temperature sensor, by means of which the apparatus measures the internal temperature of the oven of the heating means 2. If the temperature starts to decrease owing to the cold deep-frozen portion, the apparatus infers that a food portion is in the oven and starts automatically to warm the food portion in connection with the provision of the food portion according to the heating instruction it receives, simultaneously monitoring the internal temperature of the oven by means of the temperature sensor.

Also a number of food portions can be heated with the oven at the same time. The system is able to change the heating program according to need.

If a food portion 14 is not detected in the oven of the heating means 2, i.e. the temperature in the oven does not decrease sufficiently, the apparatus does not start the heating function at all. In this case the apparatus of the user detects a malfunction, in which the food portion 14 is removed from the cold space 1 of the apparatus, but it is not put into the oven of the heating means 2. This is registered as an event in the total system, from where information can be transmitted onwards to the system server 5. Also in this case the apparatus of the user, among other things, prompts with speech to put the food portion 14 into the oven and gives other instructions. This is a good safety solution e.g. for demented people.

When the food portion 14 is heated and ready, the control system 3 of the apparatus notifies this in speech, in which case the food portion 14 can be moved out of the oven of the heating means 2 by hand.

In the arrangement according to the invention different services, including telecommunication connections, e.g. via the Internet, are produced for the apparatus of the user. The services comprise e.g. various alarms when the user does something wrong. An example that can be given in this case is that the customer has not placed the food portion into the oven or has not taken a meal by a certain time. Other necessary alarms can also be given. Additionally, the providers of a service or e.g. relatives provided with user passwords and user rights can record reminders and send them via the Internet to the apparatus of the user and tell in the user interface of the browser of their own computer at what time and on what day the reminder is to be sent and also whether the reminder is of a non-recurring or recurring nature. Reminders sent as speech messages can concern catering, the taking and dosage of medicines and other necessary matters. The reminder and the instructions must be acknowledged via the pushbutton 21 of the apparatus of the user. Additionally, the apparatus of the user is provided with means, by the aid of which it also functions as a media means, such as e.g. a radio, to which a program can be sent via the Internet. Likewise, e.g. bingo-playing and corresponding entertainment games can be sent via the apparatus.

It is essential to the arrangement according to the invention that the food portion 14 and its heating instruction are paired with each other such that the apparatus knows which food portion 14 is on which shelf 13 and which heating instruction is connected to which food portion 14. This is important because the heating instructions of different food portions differ considerably from each other, in which case only with correctly performed heating is it possible to receive good-quality home-cooked food as the end result.

It is obvious to the person skilled in the art that the invention is not limited to the embodiment example presented above, but that it may vary within the scope of the claims to be presented below. Thus, for example, the arrangement is described above as a separate apparatus, but the arrangement can also be implemented as an integrated structure, in which the cold-storage space, the heating means, the user interface, the alarm system and the control system form a single domestic appliance entity.

It is also obvious to the person skilled in the art that the control system can be placed somewhere else than in connection with the cold-storage space, it can be placed for example in connection with the heating means.

It is also obvious to the person skilled in the art that the spring-like sensing means on the shelf that is presented can be structurally different to what is described above. It can be a rod, or corresponding, provided with a spring, which pushes a food portion towards the rear end of the shelf when the food portion is placed onto the shelf. In this case a detent on the rear end of the rod triggers the switch on the rear wall of the shelving structure in the same way as the presser part of the spring-like sensing means when placing a shelf back into its position. When there is no food on the shelf the spring pushes the rod forwards, as far as the point where the detent of the rod triggers the switch when placing an empty shelf back into its position. The sensing means can thus be any structure whatsoever that detects a food portion on the shelf and that also identifies into which shelf space the shelf is being placed.

It is also obvious to the person skilled in the art that the name, heating instruction and other possible identification information of a food portion can be given to the control system of the apparatus of the user in another manner than by means of a memory stick connected to the USB connector in connection with the input of a food portion. All the identification information together with the names and heating instructions can be e.g. pre-entered in the memory of the control system beforehand. In this case only updating of the information in the memory when new food portions, which have not previously been used in the arrangement, come into use is sufficient. Correspondingly, all the identification information together with the names and heating instructions of food portions can also be pre-entered in advance in the server of the service provider, from where they are downloaded in connection with the input of a food portion, e.g. via the Internet or other suitable data transfer network or communication network, to the apparatus of the user.

It is further obvious to the person skilled in the art that the identification of a food portion can be performed in different way than in connection with taking from the shelf.

A food portion can be identified e.g. in connection with placement in the oven using RFID technology or using some other corresponding identification technology. In this case the heating instruction corresponding to the latest food portion is given to the control system.

It is further obvious to the person skilled in the art that the alarms, reminders and instructions in the arrangement can be sent also by text messages and by e-mail.

Furthermore, it is obvious to the person skilled in the art that instead of a barcode system the identification of food portions can be based on another solution, such as on a magnetic stripe or corresponding, by means of which the markings about food portions brought to the cold-storage space are made adequately clear.

It is further obvious to the person skilled in the art that the alarm system can also be different from the embodiment example presented above. For example, the alarm device can be located somewhere else than around the wrist or it can be an automated device.

It is also obvious to the person skilled in the art that the microphone can also be attached to the alarm device and also a separate loudspeaker can be attached to the alarm device.

It is further obvious to the person skilled in the art that the alarm system can also be simpler than the embodiment example presented above. For example, the alarm system can comprise only the alarm device and the necessary means to send an alarm to the service center.

It is yet further obvious to the person skilled in the art that the alarm system can comprise means to localize the situation of the portable alarm device. In that case the person wearing the portable alarm device in his/her wrist can be quickly and easily found also in a large and complicated building.

## Claims

1. Arrangement for nutrition and care services, which arrangement comprises at least a cold-storage space (1) of food portions (14) together with a storage means (13) of the food portions (14); and a heating means (2); means (4a, 4b, 10, 28) for identifying the name, heating instruction and a placement location of a food portion (14) disposed in the cold-storage space (1) in connection with placing the food portion (14) in the cold-storage space (1); and means for pairing the food portion (14) and its heating instruction with each other so that the apparatus knows which food portion (14) is on which shelf (13) and which heating instruction is connected to which food portion (14); and a control system (3) provided with a user interface (4) which control system (3) is connected to a public telecommunication network, **characterized in that** the arrangement comprises an alarm system (35) for handling alarms in cases of emergency, which alarm system (35) is connected to the same control system (3) with the cold-storage space (1) and heating means (2).

2. Arrangement according to claim 1, **characterized in that** the control system (3) is connected to the cold-storage space (1), heating means (2) and alarm system (35) in a way that makes it possible to use the same food handling apparatus for food processing and for sending an emergency alarm and other information, and for communicating questions and/ or instructions to the person using the alarm system (35).

3. Arrangement according to claim 1 or 2, **characterized in that** the alarm system (35) comprises at least a portable wireless alarm device (36) and/or a wired or wireless sensor unit (37), which are connected to the control system (3), and which alarm system (35) comprises also a microphone (38) and has means to communicate with an external service center (39) through the control system (3) and a public telecommunication network.

4. Arrangement according to claim 1, 2 or 3, **characterized in that** the alarm system (35) and the control unit (3) has bi-directional self-diagnostic malfunction means to monitor the alarm devices (36) wirelessly or via wireline in order, for instance, to keep track of the battery life of the alarm device (36), and to send an error message to the control system (3) if any malfunctioning is noticed in the devices of the alarm system (35), for instance in the alarm device (36) .

5. Arrangement according to any of the preceding claims, **characterized in that** the storage means (13) of the food portions (14) comprises essentially mechanical sensing means (28) for identifying the presence of a food portion (14) in the storage means (13), and **in that** the cold-storage space (1) comprises switches (10) in connection with the control system (3) that are situated for each shelf space (9a) to be received by the storage means (13), which switches are fitted to detect on the basis of the position of the sensing means (28) whether a storage means (13) placed into the cold-storage space (1) contains a food portion (14) or not, and that the rear part of the sensing means (28) comprises a presser element (30), which is fitted to trigger the switch (10) disposed in connection with the shelf space (9a) when a food portion (14) is in the storage means (13).

6. Arrangement according to any of the preceding claims, **characterized in that** the cold-storage space (1) and the heating means (2) are separate units, which comprise contact means, by the aid of which the cold-storage space (1) and the heating means (2) are fitted to communicate between themselves either wirelessly or via wireline.

7. Arrangement according to any of the preceding claims, **characterized in that** one or more cold-storage spaces (1), one or more heating means (2) and, when necessary, one or more alarm systems (35) are attached to the same control system (3).

8. Arrangement according to any of the preceding claims, **characterized in that** the heating means (2) is provided with a loudspeaker (6), and **in that** the arrangement comprises means for informing about the preparation of the heating of a food portion (14) and about other matters for notification, communicating in alarm situations or reminding by speech via the loudspeaker (6), and that the arrangement comprises means for recording reminders and instructions in speech message format and for sending the aforementioned reminders and instructions, as well as e.g. radio programs and entertainment programs, such as games, via the Internet or corresponding telecommunication network to the apparatus of the user.

9. Arrangement according to any of the preceding claims, **characterized in that** the arrangement comprises an iButton reader (22) and escort memories, into each of which some code is programmed, with which commands are transmitted to the control system (3) and via the control system (3) to the system server (5), for listening to e.g. a voice mailbox or radio broadcasts or for sending a call request, in which case the system is fitted e.g. to send a call request by text message to a pre-defined phone number.

10. Method for nutrition and care services for processing food portions in an apparatus having at least a control system (3) and a user interface (4), in which method food portions (14) are disposed in a cold-storage space (1) for storage and heated with a heating means (2) for heating, and in which method a placement location of the food portion (14) in the cold-storage space (1) is identified mechanically by means of switches (10) and sensor means (28), and in which method a heating instruction and name of each food portion (14) are identified by means of the user interface (4) and are sent to the control system (3) of the apparatus, and the food portion (14) and its heating instruction are paired with each other so that the apparatus knows which food portion (14) is on which placement location and which heating instruction is connected to which food portion (14), and the food portion (14), **characterized in that** in addition to only handling of food portions in the apparatus, emergency alarms are handled in the same apparatus with alarm means (36-38) and communicated further through the control system (3) via the same telecommunication connections as other service information concerning the food portions (14).

11. Method according to claim 10, **characterized in that** the alarm is received wirelessly from the portable alarm device (36) and sent to an external service center (39) through the control system (3) of the apparatus.

12. Method according to claim 10 or 11, **characterized in that** the functions, for example battery life of the alarm device (36) and/or the sensor unit (37) is monitored by the monitoring means of the alarm system (35), and that through a bi-directional self-diagnostic malfunction monitoring system error messages are sent from the alarm system (35), for example from the alarm device (36) and/or from the sensor unit (37) to the control system 3.

13. Method according to any of claims 10-12 above, **characterized in that** the heating instruction of each food portion (14) is identified at the latest before the food is heated.

14. Method according to any of claims 10-13 above, **characterized in that** different services are produced for the apparatus of the user via, among other things, telecommunication connections, e.g. the Internet, which services comprise e.g. alarms given in speech or with sound signals or light signals when the user does something wrong, and also reminders to be sent as speech messages, which can concern catering, the taking and dosage of medicines and other necessary matters, which reminders are acknowledged by means of a pushbutton (21).

15. Method according to any of claims 10-14 above, **characterized in that** a radio program and entertainment games intended to be played by the user are sent to the apparatus of the user via telecommunication connections.

## Patentansprüche

1. Einrichtung für einen Nahrungsmittelversorgungs- und Fürsorgedienst, welche Einrichtung mindestens einen Kühlspeicher-Raum (1) für Essensportionen (14) zusammen mit Lager-Mitteln (13) zu den Essensportionen (14) aufweist; ferner eine Heizeinrichtung (2), Mittel (4a, 4b, 10, 28) zum Identifizieren von einem Namen, von Aufwärm-Instruktionen und von einer Platzierungsstelle einer Essensportion (14), wie sie in dem Kühlspeicher-Raum (1) hinterlegt ist, in Verbindung mit einer Platzierung der Essensportion (14) in dem Kühlspeicher-Raum (1); und mit Mitteln zum Kombinieren der Essensportion (14) und deren Aufwärminstruktion zueinander, so dass die Apparatur weiß, welche Essensportion (14) sich auf welchem Tablett (13) befindet und welche AufwärmInstruktion mit welcher Essensportion (14) in Verbindung steht; sowie ein Steuersystem (3) versehen mit einer Anwender-Schnittstelle (4), welches Steuersystem (3) an ein öffentliches Telekommunikations-Netzwerk angeschlossen ist, **dadurch gekennzeichnet, dass** die Einrichtung ein Alarmsystem (35) zum Handhaben eines Alarms im Falle eines Zwischenfalles aufweist, welcher Alarmsystem (35) an dasselbe Steuersystem (3) mit dem Kühlspeicher-Raum (1) und der Aufwärm-Vorrichtung (2) angeschlossen ist.

2. Einrichtung gemäß Anspruch 1, **dadurch gekennzeichnet, dass** das Steuersystem (3) an den Kühlspeicher-Raum (1), die Aufwärm-Vorrichtung (2) und das Alarmsystem (35) in einer Weise angeschlossen ist, die es ermöglicht, dieselbe Essens-Handhabungsvorrichtung zum Zubereiten von Essen zu nutzen und um einen Zwischenfall-Alarm und andere Information zu senden, sowie zum Kommunizieren von Fragen und/oder Instruktionen an die das Alarmsystem (3) nutzenden Person.

3. Einrichtung gemäß Anspruch 1 oder 2, **dadurch gekennzeichnet, dass** das Alarmsystem (35) zumindest eine tragbare drahtlose Alarmvorrichtung (36) und/oder eine verkabelte oder kabellose Sensoreinheit (37) aufweist, die an das Steuersystem (3) angeschlossen ist/sind, und welches Alarmsystem (35) auch ein Mikrofon (38) aufweist, und Mittel hat, um mit einem externen Servicecenter (39) durch das Steuersystem (3) und einem öffentlichen Telekommunikations-Netzwerk zu kommunizieren.

4. Einrichtung gemäß Anspruch 1, 2 oder 3, **dadurch gekennzeichnet, dass** das Alarmsystem (35) und die Steuereinheit (3) Mittel für eine bidirektionale, selbstdiagnostizierende Fehlfunktion zum Anzeigen der Alarmvorrichtungen (36) entweder drahtlos oder über eine Drahtleitung aufweist, um beispielsweise die Batterielebensdauer der Alarmvorrichtung (36) nachverfolgen zu können, und um eine Fehlermeldung an das Steuersystem (3) abzusenden, falls eine Fehlfunktion in den Vorrichtungen des Alarmsystems (35) vermerkt wird, beispielsweise in der Alarmvorrichtung (36).

5. Einrichtung gemäß einem der vorangehenden Ansprüche, **dadurch gekennzeichnet, dass** die Lagermittel (13) der Essensportionen (14) eine im Wesentlichen mechanische Sensorvorrichtung (28) zum Identifizieren des Vorliegens einer Essensportion (14) in den Speichermitteln (13) aufweist, und dass der Kühlspeicher-Raum(1) Schalter (10) in Verbindung mit dem Steuersystem (3) aufweist, die für jeden Ablageraum (9a) platziert sind, wie sie von jedem Lagermittel (13) aufgenommen sind, welche Schalter dazu bestimmt sind, auf der Grundlage der Position der Sensormittel (28) zu detektieren, ob in einem in den Kühlspeicher-Raum (1) platzierten Lagermittel (13) eine Essensportion (14) enthalten ist oder nicht, und dass der rückwärtige Teil der Sensor-Mittel (28) ein Drückerelement (30) aufweist, das dazu vorgesehen ist, den in Verbindung mit dem Ablageraum (9a) vorgesehenen Schalter (10) zu triggern, wenn eine Essensportion (14) in dem Lagermittel (13) vorhanden ist.

6. Einrichtung gemäß einem der vorangehenden Ansprüche, **dadurch gekennzeichnet, dass** der Kühlspeicher-Raum(1) und die Aufwärm-Vorrichtung (2) separate Einheiten sind, die Kontaktmittel aufweisen, mit deren Hilfe der Kühlspeicher-Raum (1) und die Aufwärmvorrichtung (2) zur Kommunikation zwischen ihnen entweder drahtlos oder über eine Drahtleitung befähigt sind.

7. Einrichtung gemäß einem der vorangehenden Ansprüche, **dadurch gekennzeichnet, dass** ein oder mehrere Kühlspeicher-Räume (1), eine oder mehrere Aufwärmvorrichtungen (2), und bei Bedarf ein oder mehrere Alarmsysteme (35) an demselben Steuersystem (3) anliegen.

8. Einrichtung gemäß einem der vorangehenden Ansprüche, **dadurch gekennzeichnet, dass** die Aufwärmvorrichtung mit einem Lautsprecher (6) versehen ist, und dass die Einrichtung Mittel aufweist, um über die Vorbereitung der Aufwärmung einer Essensportion (14) und über andere Aspekte einer Meldung, zu informieren, eine Kommunikation in Alarmsituationen aufbauend oder zur Erinnerung über eine gesprochene Stimme via dem Lautsprecher (6), und dass die Einrichtung Mittel zum Aufnehmen von Erinnerungen und Instruktionen in einem Sprachnachrichtenformat und zum Senden der erwähnten Erinnerungen und Instruktionen aufweist, wie auch beispielsweise Radio- und Unterhaltungsprogramme, wie beispielsweise Spiele, abrufbar über das Internet oder ein entsprechendes Telekommunikationsnetzwerk an die Apparatur des Anwenders.

9. Einrichtung gemäß einem der vorangehenden Ansprüche, **dadurch gekennzeichnet, dass** die Einrichtung einen iButton-Leser (22) und Escort-Speicher aufweist, in denen jeweils ein Programmcode programmiert ist, mit denen Befehle an das Steuersystem (3) und über das Steuersystem (3) an den Systemserver (5) übertragen werden, um beispielsweise einer Stimmen-Mailbox oder Radiosendern zuzuhören oder einen Ruf abzusetzen, in welchem Falle das System dazu bestimmt ist, beispielsweise eine Rufanforderung durch Textnachricht an eine vordefinierte Telefonnummer zu senden.

10. Verfahren zur Handhabung von Nahrungs- und Versorgungsgegenständen zum Zubereiten von Essensportionen in einer Apparatur mit zumindest einem Steuersystem (3) und einer Anwender-Schnittstelle (4) in welchem Verfahren Essensportionen (14) in einen Kühlspeicher-Raum(1) zum Lagern abgelegt werden und mit einer Aufwärm-Vorrichtung (2) zum Erwärmen aufgewärmt werden, und in welchem Verfahren eine Platzierungsstelle der Essensportion (14) in dem Kühlspeicher-Raum mechanisch mit Hilfe von Schaltern (10) und Sensormitteln (28) identifiziert wird, und in welchem Verfahren eine Aufwärmanweisung und ein Name einer jeden Essensportion (14) mit Hilfe der Anwenderschnittstelle (4) identifiziert und an das Steuersystem (3) der Apparatur gesendet werden, und die Essensportion (14) und deren Aufwärm-Anweisung miteinander gekoppelt werden, so dass die Apparatur weiß, welche Essensportion (14) sich auf welcher Platzierungsstelle befindet und welche Aufwärmanweisung an welche Essensportion (14) gerichtet ist, und die Essensportion (14) selbst kennt, **dadurch gekennzeichnet, dass** zusätzlich zum eigentlichen Handhaben der Essensportionen in der Apparatur Zwischenfall-Alarme in derselben Apparatur mit Alarmmitteln (36-38) gehandhabt werden, und diese ferner durch das Steuersystem (3) über dieselben Telekommunikationsverbindungen wie auch andere Serviceinformation die Essensportionen (14) betreffend kommuniziert werden.

11. Verfahren gemäß Anspruch 10, **dadurch gekennzeichnet, dass** der Alarm von der tragbaren Alarmvorrichtung (36) drahtlos empfangen wird und an einen externen Servicecenter (3) durch das Steuersystem (3) der Apparatur gesendet wird.

12. Verfahren gemäß Anspruch 10 oder 11, **dadurch gekennzeichnet, dass** die Funktionen, wie z. B. die Batterielebensdauer der Alarmvorrichtung (36) und/oder der Sensoreinheit (37) durch die Überwachungsmittel des Alarmsystems (35) überwacht werden, und dass durch ein bidirektionales, selbstdiagnostizierendes Fehlfunktions-Überwachungssystem Fehlermeldungen von dem Alarmsystem (35) gesendet werden, z. B. von der Alarmvorrichtung (36) und/oder von der Sensoreinheit (37) an das Steuersystem (3).

13. Verfahren gemäß einem der obigen Ansprüche 10 bis 12, **dadurch gekennzeichnet, dass** die Aufwärmanweisung einer jeden Essensportion (14) spätestens identifiziert wird, bevor das Essen aufgewärmt wird.

14. Verfahren gemäß einem der obigen Ansprüche 10 bis 13, **dadurch gekennzeichnet, dass** unterschiedliche Services für die Apparatur des Anwenders zur Verfügung gestellt werden, beispielsweise u. a. Telekommunikationsverbindungen, wie beispielsweise das Internet, welche Services z. B. Alarme umfassen, die in Sprache oder mit Soundsignalen oder Lichtsignalen wiedergegeben werden, wenn der Anwender einen Fehler begeht, und ebenso Erinnerungen, die als Sprachnachrichten versendet werden, die ein Catering, die Einnahme und Dosierung von Medizin und anderen notwendige Schritte umfassen können, welche Erinnerungen mit Hilfe eines Druckknopfes (21) bestätigt werden.

15. Verfahren gemäß einem der obigen Ansprüche 10 bis 14, **dadurch gekennzeichnet, dass** ein Radioprogramm und Unterhaltungsspiele, wie sie durch den Anwender zu Spielen angedacht sind, auf die Apparatur des Anwenders über Telekommunikationsverbindungen gesendet werden.

## Revendications

1. Agencement pour des services de soins et de nutrition, lequel agencement comprend au moins un espace de stockage frigorifique (1) de portions d'alimentaires (14) conjointement à un moyen de stockage (13) des portions alimentaires (14) ; et un moyen de chauffage (2) ; des moyens (4a, 4b, 10, 28) pour identifier le nom, des instructions de chauffage et un emplacement de positionnement d'une portion alimentaire (14) disposée dans l'espace de stockage frigorifique (1) en relation avec la mise en place de la portion alimentaire (14) dans l'espace de stockage frigorifique (1) ; et des moyens pour apparier la portion alimentaire (14) et son instruction de chauffage l'une à l'autre de manière que l'appareil connaisse quelle portion alimentaire (14) est sur quelle étagère (13) et quelle instruction de chauffage est en connexion avec quelle portion alimentaire (14) ; et un système de commande (3) muni d'une interface utilisateur (4), lequel système de commande (3) est connecté à un réseau public de télécommunication, **caractérisé en ce que** l'agencement comprend un système d'alarme (35) pour gérer des alarmes dans des cas d'urgence, lequel système d'alarme (35) est connecté au même système de commande (3) avec l'espace de stockage frigorifique (1) et le moyen de chauffage (2).

2. Agencement selon la revendication 1, **caractérisé en ce que** le système de commande (3) est connecté à l'espace de stockage frigorifique (1), au moyen de chauffage (2) et au système d'alarme (35) d'une manière qui rend possible l'utilisation du même appareil de gestion alimentaire pour le traitement d'aliments et pour l'envoi d'une alarme d'urgence et d'autres informations, et pour la communication de questions et/ou d'instructions à la personne utilisant le système d'alarme (35).

3. Agencement selon la revendication 1 ou 2, **caractérisé en ce que** le système d'alarme (35) comprend au moins un dispositif d'alarme sans fil portable (36) et/ou une unité de détection câblée ou sans fil (37), qui sont connectés au système de commande (3), et lequel système d'alarme (35) comprend également un microphone (38) et comporte des moyens pour communiquer avec un centre de services externe (39) par le biais du système de commande (3) et d'un réseau public de télécommunication.

4. Agencement selon la revendication 1, 2 ou 3, **caractérisé en ce que** le système d'alarme (35) et l'unité de commande (3) ont des moyens d'autodiagnostic de dysfonctionnement bidirectionnels pour surveiller les dispositifs d'alarme (36) sans fil ou par le biais d'une ligne câblée de manière, par exemple, à effectuer un suivi de la durée de vie de la batterie du dispositif d'alarme (36) et à envoyer un message d'erreur au système de commande (3) si un quelconque dysfonctionnement est relevé dans les dispositifs du système d'alarme (35), par exemple dans le dispositif d'alarme (36).

5. Agencement selon l'une quelconque des revendications précédentes, **caractérisé en ce que** le moyen de stockage (13) des portions alimentaires (14) comprend essentiellement des moyens de détection mécaniques (28) pour identifier la présence d'une portion alimentaire (14) dans le moyen de stockage (13) et **en ce que** l'espace de stockage frigorifique (1) comprend des commutateurs (10) en connexion avec le système de commande (3), qui sont situés pour chaque espace d'étagère (9a) pour être reçus par le moyen de stockage (13), lesquels commutateurs sont équipés pour détecter, sur la base de la position des moyens de détection (28), si un moyen de stockage (13) placé dans l'espace de stockage frigorifique (1) contient ou non une portion alimentaire (14), et **en ce que** la partie arrière des moyens de détection (28) comprend un élément de pression (30), qui est équipé pour déclencher le commutateur (10) disposé en relation avec l'espace d'étagère (9a) quand une portion alimentaire (14) se trouve dans le moyen de stockage (13).

6. Agencement selon l'une quelconque des revendications précédentes, **caractérisé en ce que** l'espace de stockage frigorifique (1) et le moyen de chauffage (2) sont des unités séparées qui comprennent des moyens de contact, grâce auxquels l'espace de stockage frigorifique (1) et le moyen de chauffage (2) sont équipés pour communiquer entre eux soit sans fil soit par ligne câblée.

7. Agencement selon l'une quelconque des revendications précédentes, **caractérisé en ce qu'**un ou plusieurs espaces de stockage frigorifiques (1) et un ou plusieurs moyens de chauffage (2) et, le cas échéant, un ou plusieurs systèmes d'alarme (35) sont reliés au même système de commande (3).

8. Agencement selon l'une quelconque des revendications précédentes, **caractérisé en ce que** le moyen de chauffage (2) est muni d'un haut-parleur (6) et **en ce que** l'agencement comprend des moyens pour l'information sur la préparation du chauffage d'une portion alimentaire (14) et sur d'autres questions pour la notification, la communication dans des situations d'alarme ou le rappel vocal par le biais du haut-parleur (6), et **en ce que** l'agencement comprend des moyens pour enregistrer des rappels et des instructions dans un format de message vocal et pour envoyer les rappels et instructions précités, ainsi que par exemple des programmes radio et des programmes de divertissement, tels que des jeux, par le biais d'Internet ou d'un réseau de télécommunication correspondant à l'appareil de l'utilisateur.

9. Agencement selon l'une quelconque des revendications précédentes, **caractérisé en ce que** l'agencement comprend un lecteur iButton (22) et des mémoires d'accompagnement, dans chacune desquelles est programmé un certain code, avec lequel des ordres sont transmises au système de commande (3) et par le biais du système de commande (3) au serveur de système (5), pour l'écoute par exemple d'une boîte de messagerie vocale ou de radiodiffusions ou pour l'envoi d'une demande d'appel, auquel cas le système est équipé par exemple pour envoyer une demande d'appel par message de texte à un numéro de téléphone prédéfini.

10. Procédé pour des services de soins et de nutrition pour le traitement de portions alimentaires dans un appareil ayant au moins un système de commande (3) et une interface utilisateur (4), procédé dans lequel des portions d'alimentaires (14) sont disposées dans un espace de stockage frigorifique (1) pour le stockage et sont chauffées avec un moyen de chauffage (2) pour le chauffage, et procédé dans lequel un emplacement de positionnement de la portion alimentaire (14) dans l'espace de stockage frigorifique (1) est identifié mécaniquement au moyen de commutateurs (10) et des moyens de détection (28), et procédé dans lequel une instruction de chauffage et un nom de chaque portion alimentaire (14) sont identifiés au moyen de l'interface utilisateur (4) et sont envoyés au système de commande (3) de l'appareil, et la portion alimentaire (14) et son instruction de chauffage sont appariées l'une à l'autre de manière que l'appareil connaît quelle portion alimentaire (14) est sur quel emplacement de positionnement et quelle instruction de chauffage est en connexion avec quelle portion alimentaire (14), et la portion alimentaire (14), **caractérisé en ce que**, en plus de la seule gestion de portions alimentaires dans l'appareil, des alarmes d'urgence sont gérées dans le même appareil avec des moyens d'alarme (36-38) et communiquées en outre par l'intermédiaire du système de commande (3) par le biais des mêmes connexions de télécommunication que d'autres informations de service concernant les portions alimentaires (14).

11. Procédé selon la revendication 10, **caractérisé en ce que** l'alarme est reçue sans fil à partir du dispositif d'alarme portable (36) et envoyée à un centre de services externe (39) par l'intermédiaire du système de commande (3) de l'appareil.

12. Procédé selon la revendication 10 ou 11, **caractérisé en ce que** les fonctions, par exemple une durée de vie de batterie du dispositif d'alarme (36) et/ou de l'unité de détection (37), sont surveillées par les moyens de surveillance du système d'alarme (35), et **en ce que**, par l'intermédiaire d'un système de surveillance d'autodiagnostic de dysfonctionnement bidirectionnel, des messages d'erreur sont envoyés à partir du système d'alarme (35), par exemple à partir du dispositif d'alarme (36) et/ou à partir de l'unité de détection (37), au système de commande (3).

13. Procédé selon l'une quelconque des revendications 10 à 12 précédentes, **caractérisé en ce que** l'instruction de chauffage de chaque portion alimentaire (14) est identifiée au plus tard avant que les aliments ne soient chauffés.

14. Procédé selon l'une quelconque des revendications 10 à 13 précédentes, **caractérisé en ce que** différents services sont produits pour le dispositif de l'utilisateur par le biais, entre autres, de connexions de télécommunication, par exemple Internet, lesquels services comprennent par exemple des alarmes fournies vocalement ou par des signaux sonores ou des signaux lumineux quand l'utilisateur effectue quelque chose d'erroné, et également des rappels à envoyer comme des messages vocaux, qui peuvent concerner le ravitaillement, le prise et le dosage de médicaments et d'autres questions nécessaires, lesquels rappels font l'objet d'un accusé de réception au moyen d'un bouton-poussoir (21).

15. Procédé selon l'une quelconque des revendications 10 à 14 précédentes, **caractérisé en ce qu'**un programme radio ou des jeux de divertissement destinés à être joués par l'utilisateur sont envoyés à l'appareil de l'utilisateur par le biais de connexions de télécommunication.
